# FASCICULE DE BREVET EUROPEEN

(11) **EP 1 849 451 B1**
(45) Date de publication et mention de la délivrance du brevet: **16.11.2016**
(21) Numéro de dépôt: 07300983.9
(22) Date de dépôt: 25.04.2007
(51) Int. Cl.: A61K 8/02, A61K 8/04, B32B 5/02, B32B 5/26, B32B 5/30, A61K 9/70

(54) **Structure en feuille ayant au moins une face colorée**
Blattstruktur mit mindestens einer farbigen Seite
Sheet structure with at least one coloured face

(30) Priorité: 25.04.2006 FR 0651463
(43) Date de publication de la demande: 31.10.2007
(73) Titulaire: L'Oréal, 75008 Paris (FR)
(72) Inventeur: Gueret, Jean-Louis, 75016, Paris (FR); Tugas Casanoves, Montserrat, 92100, Boulogne Billancourt (FR)
(74) Mandataire: Nony

(56) Documents cités:
- EP-A1- 0 628 300
- EP-A1- 0 933 077
- EP-A1- 1 043 018
- WO-A-03/045345
- DE-A1- 10 322 444

## Description

La présente invention concerne les structures en feuille telles que les masques ou les patchs, à appliquer sur une région du corps humain, y compris du visage.

La demande de brevet européen EP 1 129 702 divulgue une structure composite ayant une matrice adhésive située entre deux couches de support, lesquelles peuvent être constituées par des non-tissés. Une telle structure peut être mouillée avant l'utilisation puis appliquée par une face sur la peau afin de relarguer vers celle-ci au moins un actif contenu dans la matrice.

Il peut s'avérer nécessaire d'indiquer à l'utilisateur la face d'application lorsque la structure est dissymétrique.

Une solution pour ce faire peut consister à colorer l'une des couches de support.

Toutefois, les feuilles de non-tissé généralement proposées par les fabricants sont blanches et la production d'un non-tissé coloré peut s'avérer coûteuse lorsque les quantités demandées sont relativement faibles.

Il existe par conséquent un besoin pour bénéficier d'un moyen permettant de différencier les deux faces d'une structure en feuille, qui soit relativement économique et simple à mettre en oeuvre.

Le brevet européen EP 0 933 067 B1 divulgue un patch nettoyant, révélateur de l'état de la peau, comportant une matrice polymérique colorée destinée à être appliquée sur la peau.

La demande de brevet européen EP 1 043 018 A1 divulgue un patch magnétique pouvant contenir une couche colorée et comportant une matrice destinée à être appliquée sur la région à traiter.

Ces réalisations antérieures n'apportent aucune solution au problème du repérage des faces d'une structure en feuille lorsque la matrice ne vient pas directement au contact de la région à traiter mais est prise en sandwich entre deux couches d'au moins un substrat.

L'invention a pour objet une structure en feuille à appliquer sur une région du corps humain, y compris du visage, comportant :
- une matrice colorée,
- un premier substrat situé d'un premier côté de la matrice, une première face de la structure en feuille étant définie par ce premier substrat,
- un deuxième substrat situé d'un deuxième côté de la matrice opposé au premier, une deuxième face de la structure en feuille étant définie par ce deuxième substrat.

Les premier et deuxième substrats sont liés de manière permanente à la matrice. Ainsi, les premier et deuxième substrats sont présents après application sur les matières à traiter, au cours de l'utilisation de la structure. Les premier et deuxième substrats sont chacun différents d'une feuille de protection amovible.

Les premier et deuxième substrats ont des opacités différentes et l'un au moins des premier et deuxième substrats a une opacité suffisamment faible pour laisser transparaître la matrice colorée sous-jacente, de telle sorte que les première et deuxième faces de la matrice apparaissent de couleurs différentes.

L'invention permet de disposer d'un moyen facile à mettre en oeuvre pour différencier les deux faces de la structure en feuille et permettre ainsi à l'utilisateur de repérer la face d'application, laquelle est par exemple celle dont la couleur est la plus soutenue.

De plus, l'invention permet d'utiliser, si on le souhaite, comme substrats, des non-tissés conventionnels de couleur blanche, tout en donnant l'impression à l'utilisateur que l'un au moins de ces substrats est coloré, par exemple en rouge, vert, bleu, jaune, orange, rose, violet, pour ne citer que quelques teintes.

L'invention permet également de diversifier l'aspect des structures en feuille, en agissant sur la couleur de la matrice, voire de créer de nouveaux effets esthétiques en utilisant des matrices de couleur non uniforme.

Avantageusement, la matrice est colorée par l'introduction d'une dispersion de particules colorées. Cela présente l'avantage, par rapport à l'utilisation d'un colorant, de réduire le risque de dégorgement de celui-ci vers les substrats et la région traitée.

Les particules utilisées peuvent être des particules enrobées d'un revêtement extérieur non opaque, ce qui permet d'isoler de la matrice les pigments ou colorants présents au sein des particules. Le revêtement extérieur d'enrobage est par exemple un matériau polymérisable ou réticulable, notamment un polymère transparent ou translucide.

Les particules colorées peuvent comporter un coeur, lequel peut être incolore ou opaque. Le coeur est par exemple constitué par un matériau inorganique, par exemple du verre. Le coeur peut être recouvert d'une écorce colorée, par exemple une couche d'au moins un pigment ou d'un polymère coloré. L'écorce peut comporter un pigment maintenu sur le coeur à l'aide d'un liant, ce dernier étant par exemple le même polymère que celui constituant le revêtement extérieur d'enrobage.

Les particules colorées dispersées dans la matrice sont par exemple des billes de verre colorées, enrobées d'un polymère transparent. La taille moyenne des particules colorées est par exemple comprise entre 10 et 1 000 µm.

Le premier substrat peut comporter une couche d'un non-tissé dont la densité est par exemple comprise entre 20 et 100 g/m².

Le deuxième substrat peut également comporter une couche d'un non-tissé dont la densité est comprise par exemple entre 5 et 30 g/m². Le non-tissé du premier substrat peut présenter une densité supérieure à celle du deuxième substrat.

La structure en feuille, qui peut former un masque ou un patch à appliquer sur la peau, peut être conditionnée dans une enveloppe de protection.

L'invention pourra être mieux comprise à la lecture de la description détaillée qui va suivre, d'un exemple de mise en oeuvre non limitatif de celle-ci, et à l'examen du dessin annexé, sur lequel :
- la figure 1 représente de manière schématique une structure en feuille selon l'invention,
- la figure 2 est une coupe selon II-II de la figure 1,
- la figure 3 représente isolément une particule colorée dispersée dans la matrice.

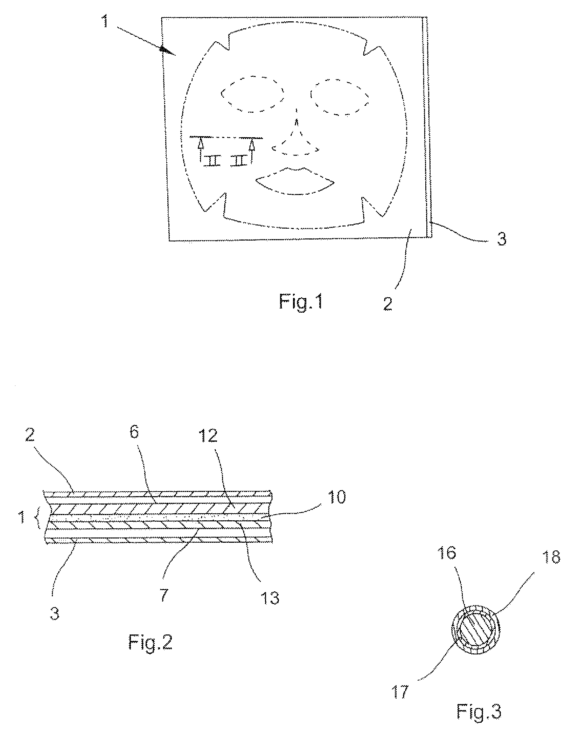

On a représenté à la figure 1 une structure en feuille 1 qui est par exemple conditionnée entre deux feuillets de protection 2 et 3, lesquels sont par exemple formés par repliement d'une bande sur elle-même.

Les feuillets 2 et 3 peuvent être constitués d'un papier ou film anti-adhérent, par exemple un papier siliconé, et peuvent venir ou non au contact des faces extérieures 6 et 7 de la structure en feuille 1. Dans un souci de clarté du dessin, un espace apparaît entre les feuillets de protection 2 et 3 et les faces 6 et 7 sur la figure 2, mais cet espace peut ne pas exister, les faces 6 et 7 pouvant le cas échéant adhérer légèrement aux feuillets 2 et 3.

La structure en feuille 1 comporte une matrice colorée 10, non blanche, disposée entre des substrats 12 et 13. La matrice 10 adhère de manière permanente aux substrats 12 et 13, ceux-ci n'étant pas prévus pour être détachés de la matrice lors de l'utilisation.

### Substrats

Conformément à un aspect de l'invention, les substrats 12 et 13 sont différents, de façon à présenter des opacités différentes et permettre aux faces extérieures 6 et 7 d'apparaître pour l'utilisateur de couleurs différentes, en laissant transparaître plus ou moins la matrice 10.

Le substrat 12 présente par exemple une épaisseur et/ou un grammage supérieur à celui du substrat 13, de façon à permettre à la face 7 d'apparaître plus colorée que la face 6.

Les substrats 12 et 13 peuvent être, en l'absence de la matrice 10, de couleur blanche.

Les substrats 12 et 13 sont de même nature, étant tous deux des non-tissés.

Les substrats 12 et 13 peuvent être monocouche ou multicouche.

### Particules colorées

La matrice 10 peut être colorée de diverses manières et avantageusement par incorporation de particules colorées enrobées.

On a représenté de manière schématique à la figure 3 un exemple d'une telle particule. Celle-ci peut comporter un coeur 16 inorganique, par exemple une bille de verre, enrobée d'une écorce colorée 17 qui est elle-même revêtue d'un revêtement d'enrobage 18 en polymère, transparent, destiné à isoler l'écorce colorée 17 de la matrice 10.

L'écorce 17 peut contenir un liant polymère qui sert à faire adhérer un pigment sur le coeur 16, par exemple un oxyde de fer ou tout autre pigment conventionnellement utilisé en cosmétique, par exemple un pigment générant une couleur par un phénomène d'absorption.

Le revêtement 18 peut être un enrobage du même polymère que celui servant de liant au sein de l'écorce 17.

Les particules colorées peuvent présenter une forme sphérique ou non.

Les particules colorées peuvent par exemple être des fibres colorées dans la masse ou des fibres enrobées d'un pigment, et éventuellement recouvertes d'un revêtement en polymère.

On peut utiliser par exemple des fibres de polyamide, de polyester, de rayonne, des fibres végétales, par exemple de coton, de maïs, de bois, de lin.

La quantité d'incorporation de particules colorées dans la matrice est liée à l'intensité de la couleur désirée et peut aller par exemple de 0,2 % en masse par rapport au poids total de la matrice sèche dans la structure en feuille, pour une couleur pastel, à 10 % en masse pour une couleur plus marquée.

Lorsque des particules enrobées sont utilisées, le revêtement extérieur des particules peut comporter un polymère choisi par exemple parmi les polymères acryliques, les cyanoacrylates, les résines urée formol, les polyuréthanes, les polymères vinyliques et les pseudo latex. Le coeur qui est enrobé peut comporter un autre matériau que le verre, par exemple choisi parmi les carbonates, la silice, le mica ou la sciure de bois.

Les particules colorées incorporées dans la matrice peuvent être sensiblement de même taille et/ou présenter des tailles différentes, afin d'obtenir des effets piquetés par exemple. Le coeur peut être réalisé dans un matériau inorganique autre que le verre ou dans une matière organique.

On peut incorporer le cas échéant, dans la matrice, des particules de couleurs différentes, afin par exemple de créer des effets de chinés et/ou de nouvelles couleurs.

### Matrice

La matrice peut être à base d'adhésif polyacrylique ou polyvinylique.

La matrice peut être hydrophobe, étant par exemple à base d'un polymère de silicone ou de polyuréthanne du type polyester polyuréthanne ou polyéther polyuréthanne.

La matrice peut encore comporter un hydrogel.

La matrice peut contenir des particules d'au moins un agent hydro-absorbant dispersées de façon homogène dans la matrice. Ces particules d'agent hydro-absorbant peuvent, en captant de l'eau, favoriser la solubilisation d'un composé actif solide hydrosoluble.

Parmi les agents hydro-absorbants pouvant être présents dans la matrice polymérique à l'état dispersé, on peut citer les polyacrylates réticulés superabsorbants à fort taux de gonflement dans l'eau, tels que ceux commercialisés par la société NORSOLOR sous la dénomination « Aquakeep^{®}»; l'alcool polyvinylique ; les polymères carboxyvinyliques tels que ceux commercialisés par la société GOODRICH sous les dénominations de « Carbopol^{®} » ; les dérivés semi-synthétiques de la cellulose tels que la carboxyméthylcellulose ; les substances naturelles telles que les amidons, les gommes naturelles (gomme de guar, gomme arabique, gomme adragante), la caséine, les phytocolloïdes (carragénates, alginates, agar-agar), les fibres de coton et la gélatine.

### Actifs

La structure en feuille peut contenir au moins un actif.

L'actif utilisé seul ou en combinaison peut être choisi parmi les actifs hydrophiles classiquement utilisés, comme les agents antioxydants, anti-radicaux libres, hydratants, dépigmentants, liporégulateurs, anti-acnéiques, antiséborrhéiques, anti-vieillissement, adoucissants, anti-rides, kératolytiques, anti-inflammatoires, rafraîchissants, cicatrisants, protecteurs vasculaires, antibactériens, antifongiques, antiperspirants, déodorants, conditionneurs de la peau, immunomodulateurs et nourrissants.

On peut utiliser notamment un ou plusieurs actifs choisis notamment parmi l'acide ascorbique et ses sels biologiquement compatibles, les enzymes, les antibiotiques tels que le phosphate de clindamycine, les composants à effet tenseur tels que les poudres de protéines de soja ou de blé, les alpha -hydroxy acides et leurs sels, les polyacides hydroxylés, les sucroses et leurs dérivés, l'urée, les aminoacides, les oligopeptides, les extraits végétaux hydrosolubles, et de levures, les hydrolysats de protéines telles que le collagène et l'élastine, l'acide hyaluronique, les mucopolysaccharides, les vitamines B₂, B₆, H, PP, le panthénol, l'acide folique, l'acide acétyl salicylique, l'allantoïne, l'acide glycyrrhétique, l'acide kojique, l'hydroquinone, etc.

La structure en feuille peut également comporter, le cas échéant, au moins un composé liposoluble choisi parmi les composés suivants : D-alpha-tocophérol, DL-alpha-tocophérol, acétate de D-alpha-tocophérol, acétate de DL-alpha-tocophérol, palmitate d'ascorbyle, vitamine F et glycérides de vitamine F, vitamines D, vitamine D₂, vitamine D₃, rétinol, esters de rétinol, palmitate de rétinol, propionate de rétinol, béta -carotène, D-panthénol, farnesol, acétate de farnesyle ; huiles de jojoba et de cassis riches acides gras essentiels ; les kératolytiques tels que l'acide salicylique, ses sels et ses esters, l'acide n-octanoyl-5 salicylique et ses esters, alkylesters d'alpha-hydroxyacides tels que l'acide citrique, acide lactique, acide glycolique ; acide asiatique, acide madécassique, asiaticoside, extrait total de centella asiatica, acide béta -glycyrrhétinique, alpha-bisabolol, céramides comme le 2 oleoylamino-1,3 octadécane ; phytanetriol, sphingomycline de lait, phospholipides d'origine marine riches en acides gras essentiels polyinsaturés, éthoxyquine ; extrait de romarin, extrait de mélisse, quercetine, extrait de microalgues séchées, anti-inflammatoires stéroïdiens.

De tels actifs peuvent être incorporés à l'état solubilisé dans des huiles, utilisées seules ou en combinaison, parmi lesquelles on peut citer : les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, de pistache, d'abricot, d'amandes ou d'avocat ; les huiles de poisson, le tricaprocaprylate de glycérol, ou les huiles végétales ou animales de formule R₁COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin ; l'huile de germes de blé, de calophyllum, de sésame, de coriandre, de carthame, l'huile de passiflore, l'huile de rosa mosqueta, de macadamia, de pépins de fruits (raisin, cassis, orange, kiwi), de colza, de coprah, d'arachide, d'onagre, de palme, de ricin, de lin, de jojoba, de chia, d'olive ou de germes de céréales comme l'huile de germes de blé, l'huile de son de riz, l'huile de karité ; des acétylglycérides; des octanoates, décanoates ou ricinoléates d'alcools ou de polyalcools ; des triglycérides d'acides gras : des glycérides ; l'huile de paraffine, de vaseline, le perhydrosqualène ; des alcools gras (alcool stéarylique, alcool cétylique) et des acides gras (acide stéarique) et leurs esters ; les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA). On peut encore citer les huiles hydrocarbonées partiellement fluorées ou les huiles perfluorées, et notamment les perfluoropolyéthers et les perfluoroalcanes. Un composé actif liposoluble peut encore être incorporé dans la matrice sous une forme pulvérulente.

### Utilisation

La structure en feuille peut être utilisée après avoir été mouillée par un liquide destiné à permettre le transfert d'au moins un actif contenu dans la matrice et/ou l'un au moins des substrats vers la peau.

Il peut s'agir d'eau, d'une solution aqueuse, d'une huile, d'une émulsion, d'un alcool, d'une solution alcoolique, entre autres.

La structure en feuille peut encore, le cas échéant, être appliquée sur une peau sèche ou mouillée. La transpiration peut suffire éventuellement, dans un exemple de mise en oeuvre, à mouiller la matrice.

### Exemple proposé

On réalise un masque pour le visage de la façon suivante.

On dépose sur un non-tissé blanc de grammage 50 g/m² une matrice colorée à l'état fluide, puis l'on couche sur celle-ci un non-tissé moins dense que l'autre, par exemple de grammage 15 g/m².

La matrice colorée peut présenter la composition suivante (% massique).
10 % ORGASOL^{®}
15 % Polyacrylate superabsorbant
40 % Glycérine
8,1 % Agent moussant
0,9 % Caféine
24 % Adhésif acrylique
particules colorées* 2 %
*Les particules colorées peuvent être obtenues comme suit.

Des billes de verre de 100 µm de diamètre sont mélangées à un pigment organique et à un liant isocyanate. Les billes ainsi enrobées sont recouvertes d'une couche de 10 µm d'isocyanate.

On obtient un masque ayant une couleur rose plus marquée sur la face servant à l'application que sur l'autre.

Ce masque peut s'utiliser en étant appliqué sur le visage après avoir mouillé avec de l'eau la face d'application.

L'expression « comportant un » doit être comprise comme étant synonyme de « comportant au moins un ».

## Revendications

1. Structure en feuille (1) à appliquer sur une région du corps humain, y compris du visage, comportant :
- une matrice colorée (10),
- un premier substrat (12) situé d'un premier côté de la matrice, une première face (6) de la structure en feuille étant définie par ce premier substrat,
- un deuxième substrat (13) situé d'un deuxième côté de la matrice, opposé au premier, une deuxième face (7) de la structure en feuille étant définie par ce deuxième substrat,
les premier et deuxième substrats ayant des opacités différentes et l'un au moins des premier et deuxième substrats ayant une opacité suffisamment faible pour laisser transparaître la matrice colorée sous-jacente, de telle sorte que les première et deuxième faces de la matrice apparaissent de couleurs différentes, les substrats (12, 13) étant des non-tissés.

2. Structure selon la revendication 1, les substrats (12, 13) étant des non-tissés de couleur blanche.

3. Structure selon l'une des revendications 1 et 2, la couleur de la matrice étant non uniforme.

4. Structure selon l'une quelconque des revendications précédentes, la matrice étant colorée par l'introduction d'une dispersion de particules colorées.

5. Structure selon la revendication 4, les particules colorées présentant un revêtement extérieur d'enrobage (18) non opaque d'un polymère transparent ou translucide.

6. Structure selon la revendication 4, les particules colorées comportant un coeur (16) recouvert d'une écorce colorée (17).

7. Structure selon la revendication 6, le coeur comportant un matériau choisi parmi le verre, les carbonates, la silice, le mica ou la sciure de bois.

8. Structure selon la revendication 6 ou 7, l'écorce (17) contenant au moins un pigment.

9. Structure selon l'une des revendications 6 à 8, le coeur étant inorganique.

10. Structure selon l'une quelconque des revendications 5 à 9, les particules colorées dispersées dans la matrice comportant des billes de verre colorées enrobées d'un polymère transparent.

11. Structure selon l'une quelconque des revendications précédentes, le premier substrat (12) comportant une couche d'un non-tissé.

12. Structure selon la revendication 11, la densité du non-tissé étant comprise entre 20 et 100 g/m².

13. Structure selon l'une quelconque des revendications précédentes, le deuxième substrat (13) comportant une couche d'un non-tissé.

14. Structure selon la revendication 13, la densité du non-tissé étant comprise entre 5 et 30 g/m².

15. Structure selon l'une quelconque des revendications précédentes, formant un masque ou un patch à appliquer sur la peau.

16. Structure selon l'une quelconque des revendications précédentes, étant conditionnée dans une enveloppe de protection (2, 3).

17. Structure selon l'une quelconque des revendications précédentes, comportant au moins un actif cosmétique.

18. Structure selon l'une quelconque des revendication précédentes, la matrice étant colorée en rouge, vert, bleu, jaune, orange, rose ou violet.

## Patentansprüche

1. Blattstruktur (1) zum Aufbringen auf einen Bereich des menschlichen Körpers, darunter das Gesicht, umfassend:
- eine farbige Matrix (10)
- ein auf einer ersten Seite der Matrix angeordnetes erstes Substrat (12), wobei eine erste Seite (6) der Blattstruktur durch dieses erste Substrat definiert ist,
- ein auf einer zweiten Seite der Matrix angeordnetes, dem ersten gegenüberliegendes zweites Substrat (13), wobei eine zweite Seite (7) der Blattstruktur durch dieses zweite Substrat definiert ist
wobei das erste Substrat und das zweite Substrat verschiedene Opazitäten aufweisen und mindestens eines des ersten und zweiten Substrats eine Opazität aufweist, die gering genug ist, um die darunterliegende farbige Matrix so durchscheinen zu lassen, dass die erste Seite und die zweite Seite der Matrix in verschiedenen Farben erscheinen, wobei die Substrate (12, 13) Vliese sind.

2. Struktur nach Anspruch 1, wobei die Substrate (12, 13) Vliese von weißer Farbe sind.

3. Struktur nach einem der Ansprüche 1 und 2, wobei die Farbe der Matrix nicht gleichmäßig ist.

4. Struktur nach einem der vorangehenden Ansprüche, wobei die Matrix durch Einbringen einer Dispersion von farbigen Teilchen gefärbt ist.

5. Struktur nach Anspruch 4, wobei die farbigen Teilchen eine äußere nicht opake Überzugsschicht (18) eines transparenten oder durchscheinenden Polymers aufweisen.

6. Struktur nach Anspruch 4, wobei die farbigen Teilchen einen Kern (16) aufweisen, der mit einer farbigen Rinde (17) bedeckt ist.

7. Struktur nach Anspruch 6, wobei der Kern ein Material umfasst, das aus Glas, Carbonaten, Siliciumdioxid, Glimmer oder Sägemehl ausgewählt ist.

8. Struktur nach Anspruch 6 oder 7, wobei die Rinde (17) mindestens ein Pigment enthält.

9. Struktur nach einem der Ansprüche 6 bis 8, wobei der Kern anorganisch ist.

10. Struktur nach einem der Ansprüche 5 bis 9, wobei die Teilchen in der Matrix dispergierten farbigen Teilchen mit einem transparenten Polymer beschichtete farbige Glaskügelchen umfassen

11. Struktur nach einem der vorangehenden Ansprüche, wobei das erste Substrat (12) eine Schicht aus einem Vlies umfasst.

12. Struktur nach Anspruch 11, wobei die Dichte des Vlieses 20 bis 100 g/m² beträgt.

13. Struktur nach einem der vorangegangenen Ansprüche, wobei das zweite Substrat (13) eine Schicht aus einem Vlies umfasst.

14. Struktur nach Anspruch 13, wobei die Dichte des Vlieses 5 bis 30 g/m² beträgt.

15. Struktur nach einem der vorangehenden Ansprüche, die eine Maske oder ein Pflaster zum Aufbringen auf die Haut bildet.

16. Struktur nach einem der vorangehenden Ansprüche, die in einer Schutzhülle (2, 3) verpackt ist.

17. Struktur nach einem der vorangehenden Ansprüche, umfassend mindestens einen kosmetischen Wirkstoff.

18. Struktur nach einem der vorangehenden Ansprüche, wobei die Matrix rot, grün, blau, gelb, orange, rose oder violett gefärbt ist.

## Claims

1. A sheet structure (1) for application to a region of the human body, including the face, the structure comprising:
- a coloured matrix (10);
- a first substrate (12) positioned on a first side of the matrix, a first surface (6) of the sheet structure being defined by this first substrate;
- a second substrate (13) positioned on a second side of the matrix, opposite the first, a second surface (7) of the sheet structure being defined by this second substrate;
the first and second substrates having different opacities, and at least one of the first and second substrates having an opacity that is sufficiently low to allow the underlying coloured matrix to show through in such manner that the first and second surfaces of the matrix appear to be of different colours, the substrates (12,13) being nonwovens.

2. The structure according to claim 1, the substrates (12, 13) being white nonwovens.

3. The structure according to one of claims 1 and 2, the colour of the matrix being non-uniform.

4. The structure according to any of the preceding claims, the matrix being coloured by dispersing coloured particles therein.

5. The structure according to claim 4, the coloured particles having a non-opaque outer coating (18) of a transparent or translucent polymer.

6. The structure according to claim 4, the coloured particles having a core (16) surrounded by a coloured shell (17).

7. The structure according to claim 6, the core comprising a material selected from among glass, carbonates, silica, mica or sawdust.

8. The structure according to claim 6 or 7, the shell (17) containing at least one pigment.

9. The structure according to one of claims 6 to 8, the core being inorganic.

10. The structure according to any of claims 5 to 9, the coloured particles dispersed in the matrix comprising coloured glass beads coated with a transparent polymer.

11. The structure according to any of the preceding claims, the first substrate (12) comprising a layer of nonwoven.

12. The structure according to claim 11, the density of the nonwoven lying in the range 20 g/m² to 100 g/m².

13. The structure according to any of the preceding claims, the second substrate (13) comprising a layer of nonwoven.

14. The structure according to claim 13, the density of the non-woven lying in the range 5 g/m² to 30 g/m².

15. The structure according to any of the preceding claims, forming a mask or a patch for application to the skin.

16. The structure according to any of the preceding claims being packaged in a protective cover (2, 3).

17. The structure according to any of the preceding claims, including at least one cosmetically active ingredient.

18. The structure according to any of the preceding claims, the matrix being coloured red, green, blue, yellow, orange, pink or purple.
